# EUROPÄISCHE PATENTSCHRIFT

(11) **EP 4 372 308 B1**
(45) Veröffentlichungstag und Bekanntmachung des Hinweises auf die Patenterteilung: **18.03.2026**
(21) Anmeldenummer: 24167999.2
(22) Anmeldetag: 16.07.2018
(51) Int. Cl.: G01N 25/72, G01N 33/32, G01B 11/06, G01B 21/02, G01B 21/08, G01B 11/02

(54) **PRÜFVERFAHREN UND PRÜFEINRICHTUNG ZUM PRÜFEN EINER OBERFLÄCHENSCHICHT**
TEST METHOD AND TEST DEVICE FOR TESTING A SURFACE LAYER
PROCÉDÉ DE CONTRÔLE ET DISPOSITIF DE CONTRÔLE POUR CONTRÔLER UNE COUCHE SUPERFICIELLE

(30) Priorität: 01.08.2017 DE 102017117398
(43) Veröffentlichungstag der Anmeldung: 22.05.2024
(62) Teilanmeldung aus: 18740835.6
(73) Patentinhaber: Dürr Systems AG, 74321 Bietigheim-Bissingen (DE)
(72) Erfinder: Ullmann, Marc, 71642 Ludwigsburg (DE)
(74) Vertreter: v. Bezold & Partner Patentanwälte - PartG mbB

(56) Entgegenhaltungen:
- EP-A1- 1 479 304
- DE-A1- 102008 048 949
- DE-C1- 19 500 073
- JP-A- 2013 134 217

## Beschreibung

Die Erfindung betrifft ein Prüfverfahren und eine entsprechende Prüfeinrichtung zum Prüfen einer Oberflächenschicht aus einem Beschichtungsmittel (z.B. lösemittelhaltiges Vorbehandlungsmittel, Primer, Klebstoff, Dichtstoff, Dämmstoff) auf einem Bauteil (z.B. Kraftfahrzeugkarosseriebauteil, Anbauteile, Komponenten, insbesondere Scheiben).

Zur Herstellung von dauerhaften Verklebungen ist häufig eine Oberflächenvorbehandlung der zu verklebenden Bauteile notwendig. Beim Verkleben von Scheiben im Automobilbau, aber auch in anderen Bereichen, wie beispielsweise dem Verkleben von lackierten Blechen oder Kunststoffen, wird derzeit eines oder eine Kombination aus folgenden Oberflächenbehandlungsverfahren verwendet.

Beispielsweise kann zum Reinigen der Oberflächen mit einem Lösungsmittel ein lösungsmittelhaltiger Filz über die Oberfläche geführt werden. Dabei werden Verschmutzungen von der Bauteiloberfläche gelöst und durch den Filz aufgenommen. Alternativ kann das Lösungsmittel auf die Oberfläche aufgebracht werden und im Nachgang mit einem Filz aufgewischt werden. In beiden Alternativen verbleibt jeweils ein dünner Film des Lösungsmittels auf der Oberfläche, der nach kurzer Zeit verdunstet.

Ein anderes Beispiel zur Oberflächenbehandlung sieht einen Aktivator vor, der zu einem sehr großen Anteil aus Lösungsmittel und zu einem sehr geringen Anteil aus einem reaktiven Material besteht. Dieser Applikator kann dann in der bereits vorstehend beschriebenen Weise mittels eines Filzes aufgebracht bzw. abgewischt werden. Auch hierbei verbleibt ein dünner Film des Aktivators auf der Bauteiloberfläche.

Bei Primern wird ein Gemisch aus Lösungsmittel, Füllstoff (z.B. Ruß) und einem reaktiven Material mit einem Filz auf die Bauteiloberfläche appliziert. Hierbei entsteht ein dünner Film auf der Oberfläche. Alternativ kann der Primer auch auf die Bauteiloberfläche aufgesprüht werden.

Die Erfindung bezieht sich nun auf die Überprüfung der Oberflächenschicht bei den oben genannten Oberflächenvorbehandlungsverfahren, wobei die Erfindung auch für andere Beschichtungsverfahren geeignet ist. Hierbei besteht in der Regel das Bedürfnis nach einer Überprüfung der Oberflächenschicht auf dem Bauteil. Hierzu sind verschiedene Methoden bekannt, die nachfolgend kurz beschrieben werden.

Eine herkömmliche Methode sieht eine Sprühstrahlüberwachung vor. Hierbei wird der Sprühstrahl des Vorbehandlungsmittels (z.B. Lösungsmittel) durch einen Sensor überwacht. Ein Nachteil dieser Methode besteht darin, dass sie beschränkt ist auf solche Verfahren, bei denen überhaupt ein Sprühstrahl appliziert wird, wohingegen eine Überprüfung der Oberflächenschicht bei einer Aufbringung mittels eines Filzes nicht möglich ist.

Eine andere bekannt Methode sieht dagegen einen Sensor zur Glanzgradkontrolle der Oberflächenschicht auf dem Bauteil vor. Hierbei wird durch einen Sensor zwischen dem Bereich mit frisch appliziertem Beschichtungsmittel (glänzend) und dem Bereich mit Siebdruck (matt) unterschieden. Nachteilig an dieser Methode ist zunächst die starke Abhängigkeit von Umgebungslicht, Abluftzeit, Beschichtungsmaterial und Oberfläche des Bauteils, so dass diese Methode nicht prozesssicher ist. Darüber hinaus kann diese Methode auch nicht als Standardverfahren eingesetzt werden, da im Vorfeld umfangreiche, projektspezifische Versuche notwendig sind.

Eine dritte Methode zur Überprüfung der Oberflächenschicht sieht dagegen eine Kameraüberwachung mit einer UV-Beleuchtung vor. Hierbei wird das Beschichtungsmittel (z.B. Primer) mit einem fluoreszierenden Material versetzt, das dann in einem abgedunkelten Bereich einer Lackierkabine mit einer Kamera detektiert werden kann. Nachteilig an dieser Methode ist zunächst die Notwendigkeit, dass das Beschichtungsmittel (z.B. Primer) mit einem fluoreszierenden Material versetzt werden muss. Dadurch wird die Materialauswahl stark eingeschränkt und die Kosten für das Beschichtungsmittel steigen stark an. Darüber hinaus muss die Lackierkabine komplett abgedunkelt werden, damit die Kamera das fluoreszierende Material erkennen kann.

Die vorstehend beschriebenen bekannten Verfahren zur Überprüfung der Oberflächenschicht auf einem Bauteil sind also jeweils mit spezifischen Problemen verbunden.

DE 10 2008 048 949 A1 offenbart ein Thermografieverfahren, bei dem die Verdunstungskälte einer Beschichtung gemessen wird, um beschichtete Oberflächenbereiche von unbeschichteten Oberflächenbereichen unterscheiden zu können.

Zum Stand der Technik ist auch hinzuweisen auf DE 195 00 073 C1, DE 10 2010002249 A1, EP 1 479 304 A1, DE 100 48 749 A1, DE 102012 007 559 A1, EP 0 624 789 A1, DE 10 2014 214 363 A1, JP 2013 134217 A, DE 10 2012 024367 A1, KR 2009 0070633 A, US 2016/067737A1, EP 1 479 304 A1 und DE 10 2015 209 861 A1.

Der Erfindung liegt deshalb die Aufgabe zugrunde, ein verbessertes Prüfverfahren und eine verbesserte Prüfeinrichtung zu schaffen.

Diese Aufgabe wird durch ein erfindungsgemäßes Prüfverfahren bzw. eine erfindungsgemäße Prüfeinrichtung gemäß den unabhängigen Ansprüchen gelöst.

Die Erfindung beruht auf der physikalisch-technischen Erkenntnis, dass lösungsmittelhaltige Beschichtungsmittel (z.B. Primer, Reinigungsmittel, Aktivatoren, etc.) nach der Aufbringung auf die Bauteiloberfläche zu einer lokalen Abkühlung führen, was durch das abdunstende Lösungsmittel verursacht wird. Diese Abkühlung kann ausgenutzt werden, um beschichtete Bereiche von nicht beschichteten Bereichen zu unterscheiden. Die Erfindung sieht deshalb vor, dass die durch die Oberflächenschicht verursachte Temperaturänderung gemessen wird, um die Oberflächenschicht zu prüfen. Beispielsweise kann auf diese Weise die Bahnbreite einer auf die Bauteiloberfläche aufgebrachten Beschichtungsmittelbahn ermittelt werden, da die Bauteiloberfläche im Bereich der Oberflächenbeschichtung lokal eine Abkühlung erfährt, wohingegen die Bauteiloberfläche neben der Beschichtungsmittelbahn keine solche Abkühlung erfährt.

Vorzugsweise erfolgt die Messung der Temperaturänderung mittels einer Thermografiekamera, wie sie an sich aus dem Stand der Technik bekannt ist. Derartige Thermografiekameras werden derzeit in vielen industriellen Anwendungen eingesetzt, beispielsweise im Bereich der Qualitätskontrolle und der zerstörungsfreien Werkstoffprüfung. Mit diesen bekannten Kameras ist es auch möglich, sehr geringe Temperaturunterschiede auf Bauteiloberflächen zu detektieren, wie es im Rahmen der Erfindung erforderlich ist.

In einer Variante der Erfindung wird das Temperaturmessgerät (z.B. Thermografiekamera) zusammen mit einem Applikator über die Bauteiloberfläche geführt, beispielsweise von einem mehrachsigen Roboter mit einer seriellen Roboterkinematik. Derartige Roboter sind als Lackierroboter aus dem Stand der Technik bekannt und müssen deshalb nicht näher beschrieben werden. Der Applikator gibt hierbei das Beschichtungsmittel auf die Bauteiloberfläche ab und das Temperaturmessgerät misst unmittelbar im Anschluss die resultierende Temperaturänderung auf der Bauteiloberfläche.

In einer anderen Variante der Erfindung erfolgen die Applikation der Oberflächenschicht und die Temperaturmessung dagegen in getrennten Prozessschritten. In einem ersten Prozessschritt wird dabei zunächst die Oberflächenschicht des Beschichtungsmittels auf das Bauteil aufgebracht. Hierzu kann beispielsweise - wie bei der vorstehend geschilderten ersten Variante der Erfindung - ein mehrachsiger Applikationsroboter eingesetzt werden, wie er an sich aus dem Stand der Technik bekannt ist. In einem anschließenden zweiten Prozessschritt wird dann die Temperaturänderung auf der Bauteiloberfläche von dem Temperaturmessgerät gemessen, wobei das Temperaturmessgerät baulich von dem Applikator getrennt ist.

Bei dieser zweiten Erfindungsvariante kann das Temperaturmessgerät beispielsweise ortsfest angeordnet sein. Alternativ besteht jedoch auch die Möglichkeit, dass das Temperaturmessgerät von einem mehrachsigen Messroboter über die Bauteiloberfläche des Bauteils geführt wird.

Weiterhin ist zu erwähnen, dass das Temperaturmessgerät vorzugsweise einen räumlichen Messbereich aufweist, der die gesamte Flächenausdehnung der Oberflächenschicht umfasst. Dies ist insbesondere dann sinnvoll, wenn das Temperaturmessgerät ortsfest angeordnet ist.

Es besteht jedoch alternativ auch die Möglichkeit, dass das Temperaturmessgerät einen räumlichen Messbereich aufweist, der nur einen Teil der Flächenausdehnung der Oberflächenschicht umfasst. In diesem Fall ist es in der Regel erforderlich, dass das Temperaturmessgerät über die Oberflächenschicht bewegt wird, damit das Temperaturmessgerät die Oberflächenschicht über ihre gesamte Flächenausdehnung vermessen kann.

Es wurde bereits vorstehend kurz erwähnt, dass aus der gemessenen Temperaturänderung die Bahnbreite einer auf die Bauteiloberfläche aufgebrachten Beschichtungsmittelbahn berechnet werden kann. Die Erfindung ist jedoch nicht auf die Bestimmung der Bahnbreite beschränkt. Vielmehr können aus der gemessenen Temperaturänderung auch andere Schichtkenngrößen abgeleitet werden, welche die Oberflächenschicht kennzeichnen. Beispielsweise gehören hierzu die Schichtdicke, die Oberflächenschicht und der Lösungsmittelgehalt der Oberflächenschicht. Hierfür ist es in einigen Fällen erforderlich, den zeitlichen Verlauf der Oberflächentemperatur auszuwerten, um daraus Rückschlüsse auf die Schichtdicke schließen zu können. Darüber hinaus kann dies auch eine Kalibrierung des Messsystems erforderlich machen.

In einem bevorzugten Ausführungsbeispiel der Erfindung wird die Temperatur bzw. die Temperaturänderung nicht nur an einem bestimmten Punkt der Oberflächenschicht gemessen. Vielmehr erfasst das Temperaturmessgerät (z.B. Thermografiekamera) vorzugsweise ein ausgedehntes Temperaturbild der Oberflächenschicht, so dass die Information über die Temperaturänderung an verschiedenen Stellen der Oberflächenschicht vorliegt.

Weiterhin ist zu erwähnen, dass sich die Erfindung auch besonders eignet für die Applikation von temperierten Beschichtungsmitteln. So weisen bestimmte Beschichtungsmittel (z.B. Klebstoff, Dämmstoff, Dichtstoff) in der Regel einen bevorzugten Temperaturbereich auf, der für die anschließenden Prozessschritte besonders geeignet ist. Vor der Applikation wird das Beschichtungsmittel deshalb auf eine bestimmte Temperatur gebracht, insbesondere durch eine Erwärmung. Nach der Applikation des Beschichtungsmittels wird dann im Rahmen der Temperaturmessung geprüft, ob die Temperatur des Beschichtungsmittels in der Oberflächenschicht innerhalb des bevorzugten Temperaturbereichs liegt. Der anschließende Prozessschritt (z.B. Verklebung) wird dann erst dann durchgeführt, wenn die gemessene Temperatur der Oberflächenschicht innerhalb des bevorzugten Temperaturbereichs liegt, der sich für eine Weiterverarbeitung eignet.

Darüber hinaus ermöglicht die erfindungsgemäße Temperaturmessung auch eine Erkennung von Fehlstellen (z.B. Lufteinschlüssen, Verunreinigungen) in der Oberflächenschicht auf dem Bauteil. So führen beispielsweise Lufteinschlüsse und Verunreinigungen zu einer lokalen Änderung des Temperaturbildes der Oberflächenschicht, was es ermöglicht, derartige Fehlstellen anhand einer Auswertung des Temperaturbildes zu erkennen.

Die Erfindung beansprucht nicht nur Schutz für das vorstehend beschriebene erfindungsgemäße Prüfverfahren. Vielmehr beansprucht die Erfindung auch Schutz für eine entsprechende Prüfeinrichtung mit einem geeigneten Temperaturmessgerät (z.B. Thermografiekamera).

Darüber hinaus weist die erfindungsgemäße Prüfeinrichtung in der praktischen Realisierung auch eine Auswertungseinheit auf, welche die gemessenen Temperaturdaten des Temperaturmessgerätes auswertet.

Weiterhin kann die erfindungsgemäße Prüfeinrichtung auch den Applikationsroboter umfassen, an dem sowohl der Applikator als auch das Temperaturmessgerät angebracht sein können.

Andere vorteilhafte Weiterbildungen der Erfindung sind in den Unteransprüchen gekennzeichnet oder werden nachstehend zusammen mit der Beschreibung der bevorzugten Ausführungsbeispiele der Erfindung anhand der Figuren näher erläutert. Es zeigen:
- Figur 1: eine schematische Darstellung einer erfindungsgemäßen Prüfeinrichtung gemäß einer ersten Erfindungsvariante,
- Figur 2: ein Flussdiagramm zur Erläuterung der Betriebsweise der Prüfeinrichtung gemäß Figur 1,
- Figur 3: eine Abwandlung von Figur 1 gemäß einer zweiten Erfindungsvariante,
- Figur 4: ein Flussdiagramm zur Verdeutlichung der Betriebsweise der Prüfeinrichtung gemäß Figur 3,
- Figur 5: ein Flussdiagramm zur Erläuterung der Temperaturmessung bei der Applikation von temperierten Beschichtungsmitteln, sowie
- Figur 6: ein Flussdiagramm zur Erläuterung der Erkennung von Fehlstellen in der Oberflächenschicht anhand des gemessenen Temperaturbildes.

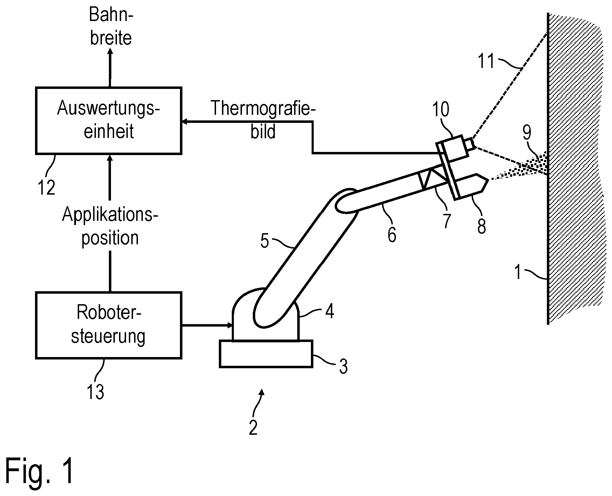

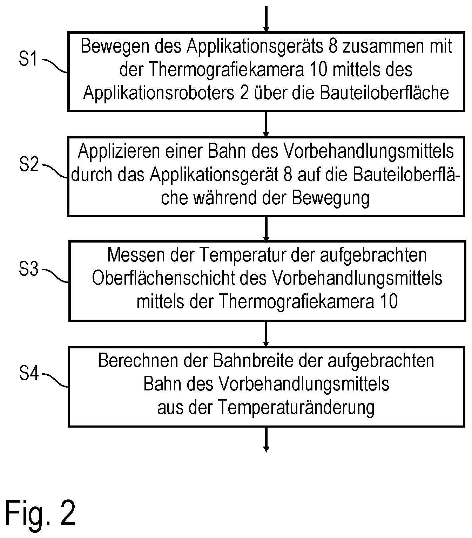

Im Folgenden wird nun das Ausführungsbeispiel gemäß Figur 1 beschrieben, wobei die Betriebsweise des Ausführungsbeispiels gemäß Figur 1 in Figur 2 als Flussdiagramm dargestellt ist.

Figur 1 zeigt eine erfindungsgemäße Prüfeinrichtung zum Prüfen einer Oberflächenschicht aus einem Beschichtungsmittel (z.B. lösungsmittelhaltiges Vorbehandlungsmittel, Primer, Klebstoff, Dichtstoff, Dämmstoff) auf ein Bauteil 1 (z.B. Kraftfahrzeugkarosseriebauteil).

Die erfindungsgemäße Prüfeinrichtung weist zunächst in Übereinstimmung mit dem Stand der Technik einen Applikationsroboter 2 auf, der weitgehend herkömmlich aufgebaut sein kann und deshalb nachfolgend nur kurz beschrieben wird.

Zunächst umfasst der Applikationsroboter 2 eine Roboterbasis 3, die wahlweise ortsfest montiert ist oder entlang einer Verfahrachse verfahrbar sein kann. Die Roboterbasis 3 trägt ein drehbares Roboterglied 4, das relativ zu der Roboterbasis 3 um eine vertikale Drehachse drehbar ist.

An dem drehbaren Roboterglied 4 ist ein proximaler Roboterarm 5 schwenkbar angebracht, wobei der proximale Roboterarm 5 um eine horizontale Schwenkachse relativ zu dem drehbaren Roboterglied 4 schwenkbar ist. Entsprechend der üblichen Roboterterminologie wird der proximale Roboterarm 5 auch als "Arm 1" bezeichnet.

Am distalen Ende des proximalen Roboterarms 5 ist ein distaler Roboterarm 6 schwenkbar angebracht, wobei der distale Roboterarm 6 an seinem distalen Ende eine mehrachsige Roboterhandachse 7 trägt.

An der Roboterhandachse 7 ist ein Applikationsgerät 8 montiert, das einen Beschichtungsmittelstrahl 9 des zu applizierenden Beschichtungsmittels auf die Oberfläche des Bauteils 1 abgibt.

Der Applikationsroboter 2 unterscheidet sich nun von herkömmlichen Applikationsrobotern dadurch, dass an der Roboterhandachse 7 zusätzlich eine Thermografiekamera 10 montiert ist. Die Thermografiekamera 10 wird von dem Applikationsroboter 2 zusammen mit dem Applikationsgerät 8 über die Bauteiloberfläche des Bauteils 1 geführt. Die Thermografiekamera 10 weist hierbei einen Messbereich 11 auf, der die gesamte Flächenausdehnung der von dem Applikationsgerät 8 auf die Oberfläche des Bauteils 1 aufgebrachten Oberflächenschicht umfasst. Bei der Applikation der Oberflächenschicht misst die Thermografiekamera 10 also unmittelbar nach der Applikation die Abkühlung in der Oberflächenschicht, die durch das Abdunsten von Lösungsmittel aus der Oberflächenschicht verursacht wird.

Die Thermografiekamera 10 ist hierbei mit einer Auswertungseinheit 12 verbunden, die das von der Thermografiekamera 10 aufgenommene Temperaturbild der Oberflächenschicht auswertet. Dabei kann die Auswertungseinheit 12 beispielsweise die Bahnbreite einer auf die Oberfläche des Bauteils 1 aufgebrachten Beschichtungsmittelbahn ermitteln. Dies wird dadurch ermöglicht, dass das Abdunsten des Lösungsmittels aus der Beschichtungsmittelbahn dort zu einer lokalen Abkühlung führt, was seitlich neben der Beschichtungsmittelbahn in den unbeschichteten Bereichen der Bauteiloberfläche nicht der Fall ist.

Darüber hinaus ist hierbei eine Robotersteuerung 13 vorgesehen, welche den Applikationsroboter 2 in bekannter Weise ansteuert. Darüber hinaus stellt die Robotersteuerung 13 der Auswertungseinheit 12 auch die aktuelle Applikationsposition zur Verfügung, d.h. den Auftreffpunkt des Beschichtungsmittelstrahls 9 auf der Oberfläche des Bauteils 1. Dies ermöglicht es der Auswertungseinheit 12, die gemessenen Temperaturwerte einem bestimmten Punkt auf der Bauteilfläche zuzuordnen.

Im Folgenden wird nun zunächst die Betriebsweise der Prüfeinrichtung 1 beschrieben, wobei auf das Flussdiagramm gemäß Figur 2 Bezug genommen wird.

In einem ersten Schritt S1 wird das Applikationsgerät 8 von dem Applikationsroboter 2 über die Oberfläche des Bauteils 1 geführt.

Dabei wird in einem Schritt S2 eine Bahn des Beschichtungsmittels (z.B. Vorbehandlungsmittel) von dem Applikationsgerät 8 auf die Bauteiloberfläche aufgebracht.

In einem Schritt S3 wird dann während der Bewegung die Temperatur der aufgebrachten Oberflächenschicht mittels der Thermografiekamera 10 gemessen.

In einem Schritt S4 berechnet dann die Auswertungseinheit 12 aus dem gemessenen Temperaturbild die Bahnbreite der aufgebrachten Beschichtungsmittelbahn.

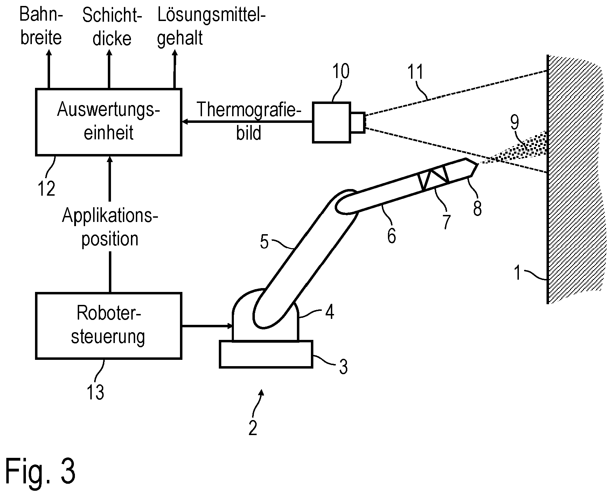

Das Ausführungsbeispiel gemäß Figur 3 stimmt weitgehend mit dem vorstehend beschriebenen Ausführungsbeispiel gemäß Figur 1 überein, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass die Thermografiekamera 10 hierbei nicht von dem Applikationsroboter 2 bewegt wird, sondern ortsfest angeordnet ist.

Darüber hinaus berechnet die Auswertungseinheit 12 hierbei aus dem aufgenommenen Thermografiebild nicht nur die Bahnbreite der auf die Oberfläche des Bauteils 1 aufgebrachten Beschichtungsmittelbahn, sondern auch die Schichtdicke der Oberflächenschicht und den Lösungsmittelgehalt.

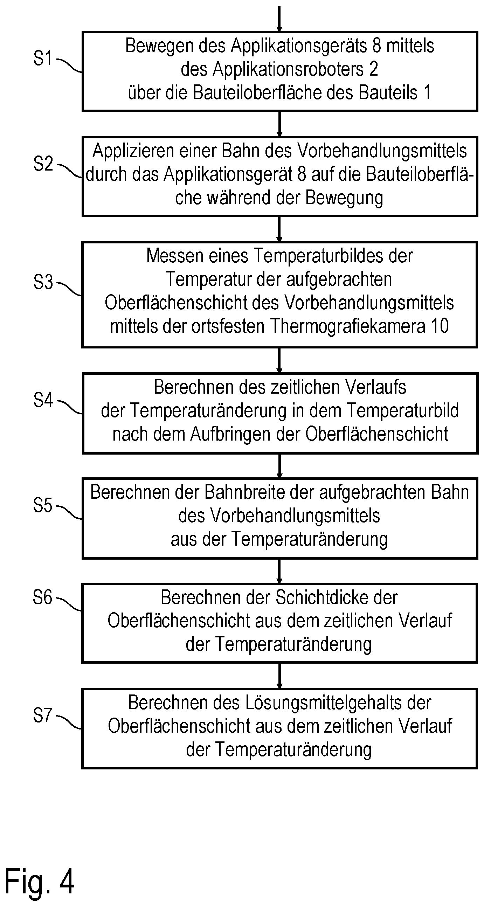

Nachfolgend wird die Betriebsweise der Prüfeinrichtung gemäß Figur 3 anhand des Flussdiagramms gemäß Figur 4 beschrieben.

In einem ersten Schritt S1 wird das Applikationsgerät 8 von dem Applikationsroboter 2 über die Oberfläche des Bauteils 1 bewegt.

Dabei wird in einem Schritt S2 eine Bahn des Vorbehandlungsmittels von dem Applikationsgerät 8 auf die Oberfläche des Bauteils 1 appliziert.

Weiterhin misst die Thermografiekamera 10 in einem Schritt S3 ein Temperaturbild der Temperatur der Oberflächenschicht auf dem Bauteil 1.

In einem Schritt S4 wird dann laufend der zeitliche Verlauf der Temperaturänderung in dem Temperaturbild berechnet.

Daraus kann dann in dem Schritt S5 die Bahnbreite der aufgebrachten Bahn des Vorbehandlungsmittels berechnet werden.

Ferner kann in einem Schritt S6 die Schichtdicke der Oberflächenschicht aus dem zeitlichen Verlauf der Temperaturänderung berechnet werden.

Schließlich kann in einem Schritt S7 auch der aktuelle Wert des Lösungsmittelgehalts in der Oberflächenschicht aus dem zeitlichen Verlauf der Temperaturänderung berechnet werden.

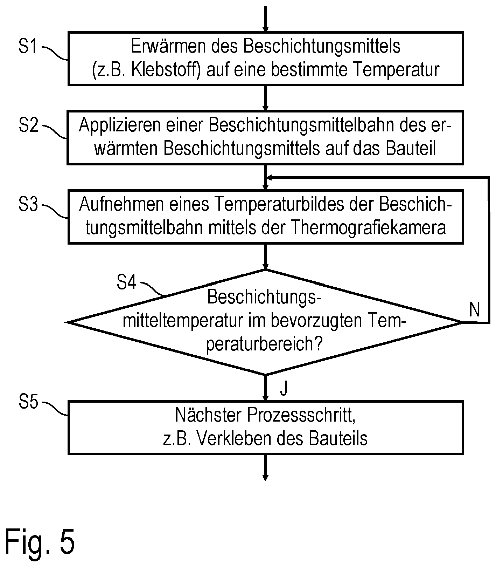

Figur 5 zeigt die Bedeutung der Temperaturmessung bei der Applikation von temperierten Beschichtungsmitteln.

Hierbei wird in einem Schritt S1 zunächst das zu applizierende Beschichtungsmittel (z.B. Klebstoff) erwärmt, um möglichst gute Applikationsbedingungen zu erreichen.

Anschließend wird das temperierte Beschichtungsmittel dann in einem Schritt S4 auf das Bauteil appliziert.

Anschließend wird dann in einem Schritt S3 ein Temperaturbild der Beschichtungsmittelbahn auf dem Bauteil aufgenommen.

Hierbei kann nach dem Aufbringen des erwärmten Beschichtungsmittels auf die Bauteiloberfläche eine Abkühlung eintreten. In einem Schritt S4 wird dann laufend geprüft, ob sich die Oberflächenschicht so weit abgekühlt hat, dass die Temperatur innerhalb eines bevorzugten Temperaturbereiches liegt, der für weitere Prozessschritte geeignet ist, beispielsweise für eine Verklebung.

Falls dies der Fall ist, so erfolgt in einem nächsten Schritt S5 der nächste Prozessschritt, beispielsweise eine Verklebung des Bauteils.

Andernfalls wird abgewartet, bis das Beschichtungsmittel hinreichend weit abgekühlt ist.

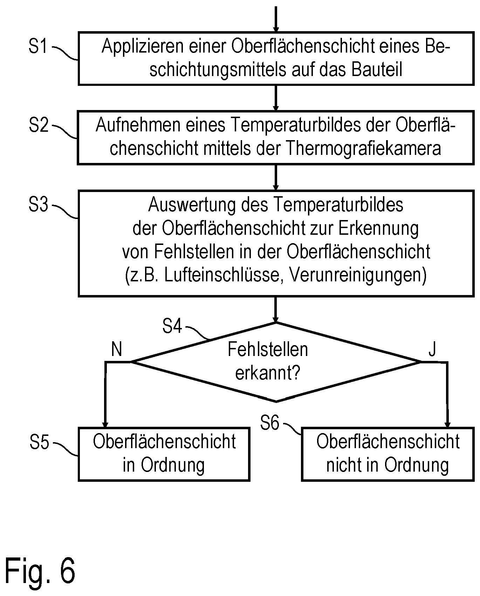

Schließlich zeigt Figur 6 ein Flussdiagramm zur Erläuterung der Erkennung von Fehlstellen (z.B. Lufteinschlüsse, Verunreinigungen) in der Oberflächenschicht durch die erfindungsgemäße Temperaturmessung.

In einem ersten Schritt S1 wird hierbei in herkömmlicher Weise die Oberflächenschicht des Beschichtungsmittels auf das Bauteil aufgebracht.

Anschließend wird in einem Schritt S2 ein Temperaturbild der Oberflächenschicht mittels der Thermografiekamera aufgenommen.

In einem Schritt S3 wird dann das Temperaturbild ausgewertet, um Fehlstellen in der Oberflächenschicht zu erkennen, wie beispielsweise Lufteinschlüsse oder Verunreinigungen. So führen derartige Fehlstellen zu einer lokal andersartigen Temperaturänderung in der Oberflächenschicht, was sich durch eine Auswertung des Temperaturbildes erkennen lässt.

In einem Schritt S4 wird dann geprüft, ob derartige Fehlstellen erkannt wurden.

Falls dies der Fall ist, so wird in einem Schritt S6 festgestellt, dass die Oberflächenschicht nicht in Ordnung ist.

Andernfalls wird in einem Schritt S5 festgestellt, dass die Oberflächenschicht in Ordnung ist.

In den beiden Schritten S5, S6 kann dann jeweils ein Fehler-Flag gesetzt bzw. gelöscht werden.

### BEZUGSZEICHENLISTE

- 1: Bauteil
- 2: Applikationsroboter
- 3: Roboterbasis
- 4: Drehbares Roboterglied
- 5: Proximaler Roboterarm ("Arm 1")
- 6: Distaler Roboterarm ("Arm 2")
- 7: Roboterhandachse
- 8: Applikationsgerät
- 9: Beschichtungsmittelstrahl
- 10: Thermografiekamera
- 11: Messbereich der Thermografiekamera
- 12: Auswertungseinheit
- 13: Robotersteuerung

## Patentansprüche

1. Prüfverfahren zum Prüfen einer Oberflächenschicht aus einem Beschichtungsmittel, insbesondere aus einem lösungsmittelhaltigen Vorbehandlungsmittel, einem Primer, einem Klebstoff, einem Dichtstoff oder einem Dämmstoff, auf einem Bauteil (1), insbesondere auf einem Kraftfahrzeugkarosserie- oder Anbaubauteil (1),
a) wobei die Oberflächenschicht nach ihrem Auftrag auf das Bauteil (1) eine Temperaturänderung verursacht, insbesondere durch Abdunsten von Lösungsmittel aus der Oberflächenschicht,
b) wobei die durch die Oberflächenschicht verursachte Temperaturänderung gemessen wird, insbesondere mittels einer Thermografiekamera (10), um die Oberflächenschicht zu prüfen,
c) wobei das Beschichtungsmittel für einen anschließenden Prozessschritt einen bevorzugten Temperaturbereich aufweist,
**dadurch gekennzeichnet,**
d) **dass** durch die Temperaturmessung geprüft wird, ob die Temperatur des Beschichtungsmittels in der Oberflächenschicht innerhalb des bevorzugten Temperaturbereichs liegt, und
e) **dass** der anschließende Prozessschritt erst durchgeführt wird, wenn die Temperatur der Oberflächenschicht innerhalb des bevorzugten Temperaturbereichs liegt.

2. Prüfverfahren nach Anspruch 1, **gekennzeichnet durch** folgenden Schritt:
Aufbringen der Oberflächenschicht auf das Bauteil (1) mittels eines Applikators (8), der das Beschichtungsmittel auf das Bauteil (1) appliziert und über die Oberfläche des Bauteils (1) geführt wird, insbesondere von einem mehrachsigen Roboter (2), wobei ein Temperaturmessgerät (10) mechanisch mit dem Applikator (8) verbunden ist und gemeinsam mit dem Applikator (8) über die Oberfläche geführt wird.

3. Prüfverfahren nach Anspruch 1, **dadurch gekennzeichnet,**
a) **dass** in einem ersten Prozessschritt die Oberflächenschicht auf das Bauteil (1) aufgebracht wird, wobei ein Applikator (8) über die Oberfläche des Bauteils (1) geführt wird, insbesondere von einem mehrachsigen Roboter (2), und
b) **dass** in einem anschließenden zweiten Prozessschritt die aufgebrachte Oberflächenschicht geprüft wird, wobei das Temperaturmessgerät (10) von dem Applikator (8) baulich getrennt ist.

4. Prüfverfahren nach einem der vorhergehenden Ansprüche, **dadurch gekennzeichnet,**
a) **dass** das Temperaturmessgerät (10)
a1) ortsfest angeordnet ist oder
a2) von einem mehrachsigen Messroboter über die Oberfläche des Bauteils (1) geführt wird,
und/oder
b) **dass** das Temperaturmessgerät (10) einen räumlichen Messbereich (11) aufweist,
b1) der die gesamte Flächenausdehnung der Oberflächenschicht umfasst, oder
b2) der nur einen Teil der Flächenausdehnung der Oberflächenschicht umfasst.

5. Prüfverfahren nach einem der vorhergehenden Ansprüche, **dadurch gekennzeichnet, dass** aus der gemessenen Temperaturänderung eine Schichtkenngröße ermittelt wird, insbesondere eine der folgenden Schichtkenngrößen:
a) Schichtdicke der Oberflächenschicht,
b) Bahnbreite der bahnförmig auf das Bauteil (1) aufgebrachten Oberflächenschicht,
c) Lösungsmittelgehalt der Oberflächenschicht.

6. Prüfverfahren nach Anspruch 5, **dadurch gekennzeichnet,**
a) **dass** die Temperaturänderung an verschiedenen Stellen der Oberflächenschicht gemessen wird, um ein räumliches Bild der Temperaturänderung zu erzeugen, und
b) **dass** aus dem räumlichen Bild der gemessenen Temperaturänderung ein entsprechendes räumliches Bild der Schichtkenngröße ermittelt wird.

7. Prüfverfahren nach einem der vorhergehenden Ansprüche, **dadurch gekennzeichnet, dass** das Beschichtungsmittel vor der Applikation auf das Bauteil (1) temperiert, insbesondere erwärmt, wird.

8. Prüfverfahren nach einem der vorhergehenden Ansprüche,
**gekennzeichnet durch** folgende Schritte:
a) Applizieren der Oberflächenschicht des Beschichtungsmittels auf das Bauteil (1),
b) Messen eines Temperaturbildes der Oberflächenschicht auf dem Bauteil (1), und
c) Auswerten des Temperaturbildes der Oberflächenschicht zur Erkennung von Fehlstellen, insbesondere Lufteinschlüssen oder Verunreinigungen, in der Oberflächenschicht.

## Claims

1. Test method for testing a surface layer consisting of a coating agent, in particular a solvent-based pretreatment agent, primer, adhesive, sealant, or insulating material, on a component (1), in particular on a motor vehicle body or attachment component (1),
a) wherein the surface layer causes a temperature change after it has been applied to the component (1), in particular due to evaporation of solvent from the surface layer,
b) wherein the temperature change caused by the surface layer is measured, in particular by means of a thermographic camera (10), in order to inspect the surface layer,
c) wherein the coating agent has a preferred temperature range for a subsequent process step,
**characterized in**
d) **that** the temperature measurement is used to check whether the temperature of the coating agent in the surface layer is within the preferred temperature range, and
e) **that** the subsequent process step is only carried out when the temperature of the surface layer is within the preferred temperature range.

2. Test method according to claim 1, **characterized by** the following step:
Applying the surface layer to the component (1) by means of an applicator (8) which applies the coating agent to the component (1) and is guided over the surface of the component (1), in particular by a multi-axis robot (2), wherein a temperature measuring device (10) is mechanically connected to the applicator (8) and is guided over the surface together with the applicator (8).

3. Test method according to claim 1, **characterized in**
a) **that** in a first process step, the surface layer is applied to the component (1), wherein an applicator (8) is guided over the surface of the component (1), in particular by a multi-axis robot (2), and
b) **that** in a subsequent second process step, the applied surface layer is inspected, wherein the temperature measuring device (10) is structurally separate from the applicator (8).

4. Testing method according to one of the preceding claims, **characterized in**
a) **that** the temperature measuring device (10)
a1) is fixed in position or
a2) is guided over the surface of the component (1) by a multi-axis measuring robot, and/or
b) **that** the temperature measuring device (10) has a spatial measuring range (11),
b1) which covers the entire surface area of the surface layer, or
b2) which only covers part of the surface area of the surface layer.

5. Test method according to one of the preceding claims, **characterized in that** a layer parameter is determined from the measured temperature change, in particular one of the following layer parameters:
a) layer thickness of the surface layer,
b) line width of the surface layer applied to the component (1) in line form,
c) solvent content of the surface layer.

6. Test method according to claim 5, **characterized in**
a) **that** the temperature change is measured at different points on the surface layer in order to generate a spatial image of the temperature change, and
b) **that** a corresponding spatial image of the layer parameter is determined from the spatial image of the measured temperature change.

7. Test method according to one of the preceding claims, **characterized in that** the coating agent is tempered, in particular heated, before application to the component (1).

8. Test method according to one of the preceding claims,
**characterized by** the following steps:
a) applying the surface layer of the coating agent to the component (1),
b) measuring a temperature image of the surface layer on the component (1), and
c) evaluating the temperature image of the surface layer to detect defects, in particular air pockets or impurities, in the surface layer.

## Revendications

1. Procédé de contrôle pour contrôler une couche de surface constituée d'un produit de revêtement, en particulier d'un produit de prétraitement contenant un solvant, d'un apprêt, d'une colle, d'un produit d'étanchéité ou d'un matériau isolant, sur un composant (1), en particulier sur un composant de carrosserie ou de montage de véhicule automobile (1),
a) la couche de surface provoquant, après son application sur le composant (1), une variation de température, en particulier par évaporation du solvant de la couche de surface,
b) la variation de température provoquée par la couche de surface étant mesurée, en particulier au moyen d'une caméra thermographique (10), afin de contrôler la couche de surface,
c) l'agent de revêtement présentant une plage de températures préférées pour une étape de traitement ultérieure,
**caractérisé en ce que**
d) la mesure de la température permet de contrôler si la température de l'agent de revêtement dans la couche de surface se situe dans la plage de températures préférée, et
e) l'étape de processus suivante n'est réalisée que lorsque la température de la couche de surface se situe à l'intérieur de la plage de températures préférée.

2. Procédé de contrôle selon la revendication 1, **caractérisé par** l'étape suivante :
Application de la couche de surface sur le composant (1) au moyen d'un applicateur (8) qui applique le produit de revêtement sur le composant (1) et qui est guidé sur la surface du composant (1), en particulier par un robot (2) à plusieurs axes, un appareil de mesure de la température (10) étant relié mécaniquement à l'applicateur (8) et étant guidé sur la surface conjointement avec l'applicateur (8).

3. Procédé de contrôle selon la revendication 1, **caractérisé en ce que**
a) dans une première étape de processus, la couche de surface est appliquée sur le composant (1), un applicateur (8) étant guidé sur la surface du composant (1), en particulier par un robot (2) à plusieurs axes, et
b) dans une deuxième étape de processus qui lui fait suite, la couche de surface appliquée est contrôlée, l'appareil de mesure de la température (10) étant séparé de l'applicateur (8) par construction.

4. Procédé de contrôle selon l'une des revendications précédentes, **caractérisé en ce que**
a) l'appareil de mesure de la température (10)
a1) est agencé de manière fixe ou
a2) est guidé par un robot de mesure à plusieurs axes sur la surface du composant (1), et/ou
b) **en ce que** l'appareil de mesure de la température (10) présente une plage de mesures spatiales (11),
b1) qui comprend la totalité de l'étendue de surface de la couche de surface, ou
b2) qui comprend seulement une partie de l'étendue de surface de la couche de surface.

5. Procédé de contrôle selon l'une des revendications précédentes, **caractérisé en ce qu'**une grandeur caractéristique de couche est déterminée à partir de la variation de température mesurée, en particulier l'une des grandeurs caractéristiques de couche suivantes :
a) épaisseur de la couche de surface,
b) largeur de bande de la couche de surface appliquée sous forme de bande sur le composant (1),
c) teneur en solvant de la couche de surface.

6. Procédé de contrôle selon la revendication 5, **caractérisé en ce que**
a) la variation de température est mesurée en différents points de la couche de surface afin de produire une image spatiale de la variation de température, et
b) une image spatiale correspondante de la grandeur caractéristique de la couche est déterminée à partir de l'image spatiale de la variation de température mesurée.

7. Procédé de contrôle selon l'une des revendications précédentes, **caractérisé en ce que** le produit de revêtement est tempéré, en particulier chauffé, avant d'être appliqué sur le composant (1).

8. Procédé de contrôle selon l'une des revendications précédentes, **caractérisé par** les étapes suivantes :
a) application de la couche de surface du produit de revêtement sur le composant (1),
b) mesure d'une image de température de la couche de surface sur le composant (1), et
c) évaluation de l'image de température de la couche de surface pour détecter des défauts, en particulier des inclusions d'air ou des impuretés, dans la couche de surface.
